# EUROPEAN PATENT APPLICATION

(11) **EP 2 832 375 A1**
(43) Date of publication of application: **04.02.2015**
(21) Application number: 14178434.8
(22) Date of filing: 24.07.2014
(51) Int. Cl.: A61L 9/12, B60H 3/00

(54) **Ambient fragrance dispenser**

(30) Priority: 01.08.2013 IT MI20131296
(71) Applicant: De Leuriks B.V., 1015 BV Amsterdam (NL)
(72) Inventor: Re, Gianpaolo, 1015 BV Amsterdam (NL)
(74) Representative: Simino, Massimo

(57) **Abstract**

An ambient fragrance dispenser comprises a body that defines a container (2) containing a first fragrancing agent, to which membrane diffuser means are applied so as to allow said first fragrancing agent to be diffused from said container (2) towards the area outside of said body. Advantageously, a first outer portion (3) of said body is impregnated with a second fragrancing agent that is diffused towards the area surrounding said body independently from the diffusion of said first fragrancing agent.

## Description

The present invention refers to an ambient fragrance dispenser in accordance with the preamble of claim 1.

According to a further aspect, the invention focuses on a package containing an ambient fragrance dispenser, as well as on a method for making an ambient fragrance dispenser and on a method for fragrancing an area.

For the sake of making the description easier, the present description is carried out, in a non-limiting manner, with particular reference to an ambient fragrance dispenser that is intended to be used inside the driver's compartment of an automobile, since the invention can also refer to different ambient fragrance dispensers, for example an ambient fragrance dispenser for domestic use.

In the field of fragrance dispensers, particularly fragrance dispensers for the driver's compartment of vehicles, there is the strong desire of having fragrance dispensers that, despite being quite small, are very efficient, said efficiency of the fragrance dispenser being evaluated based upon the duration of the fragrancing effect over time and/or the intensity of the fragrancing effect achieved.

For this purpose, it is necessary to consider that whereas the small dimensions are appreciated by any consumer, the intensity of the fragrancing effect achieved can be subjective and is highly influenced by the size of the area to be fragranced.

A further requirement is thus that of allowing the user to be able to vary the intensity of the fragrancing effect that can be obtained.

Furthermore, it is necessary to consider that different users surely appreciate the various fragrances obtained by combining various fragrances in a different manner.

A further requirement is therefore that of allowing the user to have at least small freedom in obtaining different fragrances with a same fragrance dispenser.

Concerning fragrance dispensers that are currently known, they are mainly membrane fragrance dispensers that, *per se,* despite having good performance do not make it possible to fully satisfy the aforementioned requirements.

The problem at the basis of the present invention is that of devising an ambient fragrance dispenser which has structural and functional characteristics such as to satisfy the aforementioned requirement, while at the same time avoiding the drawbacks mentioned with reference to the prior art.

Such a problem is solved with an ambient fragrance dispenser in accordance with claim 1.

According to a further aspect, such a drawback is moreover overcome with a package in accordance with claim 14, with a method for making an ambient fragrance dispenser in accordance with claim 15, as well as with a method for fragrancing an area in accordance with claim 16.

Further features and advantages of the ambient fragrance dispenser according to the present invention, as well as of the package and of the methods according to the invention shall become clearer from the following description of some preferred embodiments, given as an indication and not for limiting purposes, with reference to the attached figures, in which:
- figure 1 represents a perspective view of an ambient fragrance dispenser according to the invention equipped with a tying clip;
- figure 2 represents a perspective view of the ambient fragrance dispenser of figure 1 in accordance with a different angle;

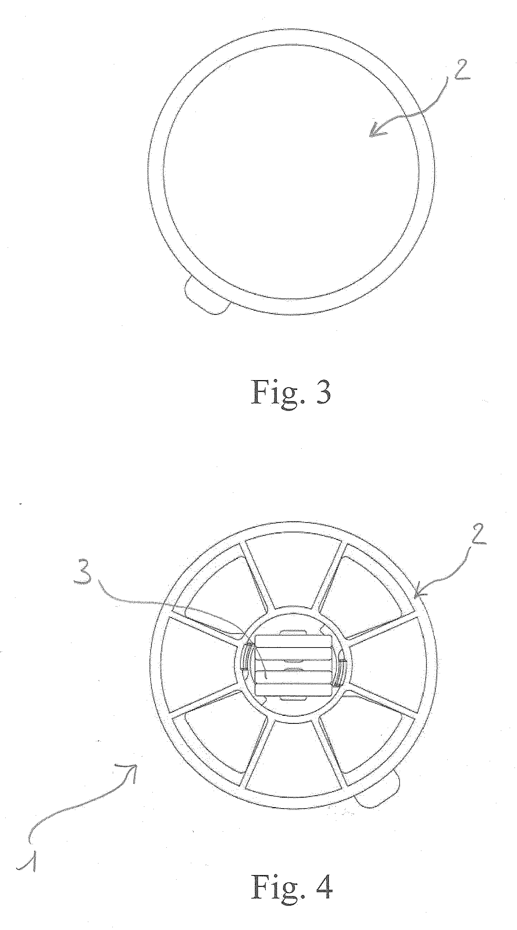

- figures 3 and 4 represent two plan views, front and rear, respectively, of the ambient fragrance dispenser of figure 1;
- figure 5 represents a side view of the ambient fragrance dispenser of figure 1;
- figure 6 represents the ambient fragrance dispenser of figure 5 with the tying clip in an exploded view;
- figure 7 represents a plan view of the ambient fragrance dispenser of figure 1;
- figure 8 represents the ambient fragrance dispenser of figure 7 with the tying clip in an exploded view;
- figure 9 represents a rear plan view of the ambient fragrance dispenser of figure 1 without the tying clip and
- figures 10, 11 and 12 represent side plan views of the ambient fragrance dispenser of figure 1 with the tying clip that is applied so as to be rotated by 90° with respect to figure 1.

With reference to the attached figures, reference numeral 1 wholly indicates an ambient fragrance dispenser according to the invention.

The ambient fragrance dispenser 1 comprises:
- a body that comprises a container 2 containing a first fragrancing agent and
- diffuser means to allow said first fragrancing agent to be diffused from the container 2 towards the area outside the body itself.

Advantageously, at least one first portion 3 of the body of the ambient fragrance dispenser 1 is impregnated with a second fragrancing agent and is in communication with the area outside of said body, so that the aforementioned second fragrancing agent can be released by the first portion 3 of the body to be diffused towards the area surrounding the body itself of the ambient fragrance dispenser 1.

It is worth highlighting that the release, i.e. the diffusion, of the second fragrancing agent from the aforementioned first portion 3 of the body of the ambient fragrance dispenser 1, towards the area surrounding the body itself, occurs in a completely independent manner with respect to the diffusion method of the aforementioned first fragrancing agent from the container 2 which, as mentioned, is carried out through the aforementioned diffuser means.

In accordance with the embodiment illustrated, the aforementioned first portion 3 of said body is an outer portion of the body of the ambient fragrance dispenser 1.

Concerning the two aforementioned fragrancing agents, it is worth highlighting that the aforementioned second fragrancing agent is different from the first fragrancing agent in composition and/or fragrance.

In particular, the single composition of each fragrancing agent is selected and optimised as a function of the specific different method with which the fragrancing agent is released and/or diffused towards the outside area. This is useful not only in terms of the components that contribute towards forming the fragrant "bouquet" but also and especially in terms of the type of carrier used for conveying the fragrant ingredients that form each specific fragrancing agent.

Furthermore, the possibility of having two separate fragrance modalities that are independent from one another available in one same fragrance dispenser, moreover based upon using two fragrancing agents having fragrances that are in any case different, makes it possible to avoid having two fragrances with the same "olfactory note", being it furthermore possible to combine the two different fragrances to obtain a new fragrance of perfume, all this being possible by using the same fragrance dispenser according to a different modality of use, that is to say with a combined use rather than separate/consecutive use of the two different fragrancing technologies implemented, as shall become clearer from the rest of the description.

In accordance with a preferred embodiment the aforementioned first portion 3 of the body of the ambient fragrance dispenser 1 comprises a polymeric material that is impregnated with said second fragrancing agent, preferably a plastic polymeric material, more preferably a plastic polymeric material comprising EVA, PVC and/or polypropylene.

In the case in which the aforementioned first portion 3 of the body of the ambient fragrance dispenser 1 is made with injection, moulding and/or hot extrusion operations of a polymer obtained from granular raw material (so-called chips), the impregnation of the polymeric material with the second fragrancing agent can advantageously be carried out by carrying out a step of impregnation of the aforementioned granular material with the second fragrancing agent, before continuing on to the melting of the granular material so as to obtain the molten polymer to be injected, moulded and/or hot extruded.

The aforementioned step of impregnation of the granular material with the second fragrancing agent can be carried out by mixing the granular material in a rotary mixer, or other mixer, in which the second fragrancing agent has been added, for example in the liquid, oil, gel state and the like.

It is worth highlighting that, the aforementioned step of impregnation of the granular material with the second fragrancing agent is obtained by exploiting the normal capability of the polymeric material of absorbing, at least in the surface layer, the fragrancing agent. The following step of making the granular polymeric material melt obtaining molten polymer causes the most impregnated polymeric material in the molten polymer to become mixed (the outermost granules) with the non-impregnated or less impregnated polymeric material (the innermost granules), obtaining a molten polymer with a greater homogeneousness of the present fragrancing agent. This makes it possible not only to homogenise the amount of second fragrancing agent in the polymeric material but moreover to have a more intimate impregnation of the polymeric material itself.

In accordance with the invention, the method for making the ambient fragrance dispenser 1 in polymeric material, comprises the steps of:
- providing a polymeric material in granular form;
- supplying and melting said granular polymeric material to obtain a molten polymer;
- by injection, moulding and/or hot extrusion of said molten polymer making one or more portions of a body of an ambient fragrance dispenser comprising a container and
- inserting in the container of said body of the ambient fragrance dispenser a first fragrancing agent.

Specifically, the aforementioned method is characterised in that the granular polymeric material intended to constitute the first portion 3 of the body is subjected to a step of perfuming with a second fragrancing agent that is different from the first fragrancing agent, so as to obtain, through injection, moulding and/or hot extrusion of said polymeric material incorporating inside it said second fragrancing agent, said first portion 3 of the body of the impregnated fragrance dispenser 1 of the second fragrancing agent.

Preferably said step of perfuming said first portion 3 of said body with said second fragrancing agent different from said first fragrancing agent is performed by impregnation of said body with second fragrancing agent.

In order to manufacture the aforementioned first portion 3 of the body of the ambient fragrance dispenser 1, as an alternative to a polymeric material it is possible to use different materials. For example, a layer of cellulose that is suitably impregnated with the second fragrancing agent.

Preferably, the aforementioned first portion 3 of the body of the ambient fragrance dispenser 1 constitutes a support, gripping, clamping and/or fastening portion of such a body.

Preferably, the aforementioned first portion 3 of the body 1 of the ambient fragrance dispenser is fixed to the remaining part of said body by connection means 4, preferably removable snap-in connection means.

Preferably, the aforementioned diffuser means are operatively associated with the container 2 and can be activated to pass from a deactivated condition, in which they prevent the diffusion of the first fragrancing agent outside the container 2, to an activated operative condition, in which they place in communication the inside of the container with the outside so as to allow the first fragrancing agent to be diffused in the environment outside the container itself.

More in detail, the aforementioned diffuser means comprise a plurality of passages to place in fluid communication the inside of the container 2 with the environment outside the container 2 itself.

Preferably, at the aforementioned passages adjustable partializing means are applied, said means being adjustable between an open position and a closed position.

Basically, by suitably acting on the aforementioned adjustable partializing means it is possible, within a certain margin, to obtain an adjustment of the amount of first fragrancing agent diffused in the surrounding area.

Preferably, the aforementioned diffuser means comprise membrane means that are permeable to the first fragrancing agent, so as to ensure a calibrated release of the first fragrancing agent in the area outside the container 2.

It is worth highlighting that the aforementioned container 2 can be advantageously made in a single piece with the body of the ambient fragrance dispenser.

However, in accordance with a different embodiment, the aforementioned container can be defined by a closed interchangeable capsule which is housed and/or supported inside the body of the ambient fragrance dispenser in a removable and/or interchangeable manner. Basically, such a capsule constitutes a refill to be replaced every time the first fragrancing agent contained in it has run out.

The first fragrancing agent contained inside the container 2 is preferably liquid, with it also being possible to foresee a first fragrancing agent that is only partially liquid or in gel, or even in the granular or powder form.

In accordance with the illustrated embodiment, the ambient fragrance dispenser 1 is a membrane-type ambient fragrance dispenser, and the aforementioned first portion 3 is an outer portion of the body of such an ambient fragrance dispenser. In a *per se* conventional manner, once it has been activated, the membrane of the fragrance dispenser 1 makes it possible to adjust the diffusion of the first fragrancing agent contained inside the container 2 towards the area outside the container itself.

The membrane, can be advantageously made with polyethylene, preferably high density polyethylene (HDPE).

In accordance with the illustrated embodiment, the aforementioned first impregnated portion 3 of the second fragrancing agent defines a support clip through which the body of the ambient fragrance dispenser 1 can be fixed to a support, for example to an air-vent blade of an automobile or of a conditioner.

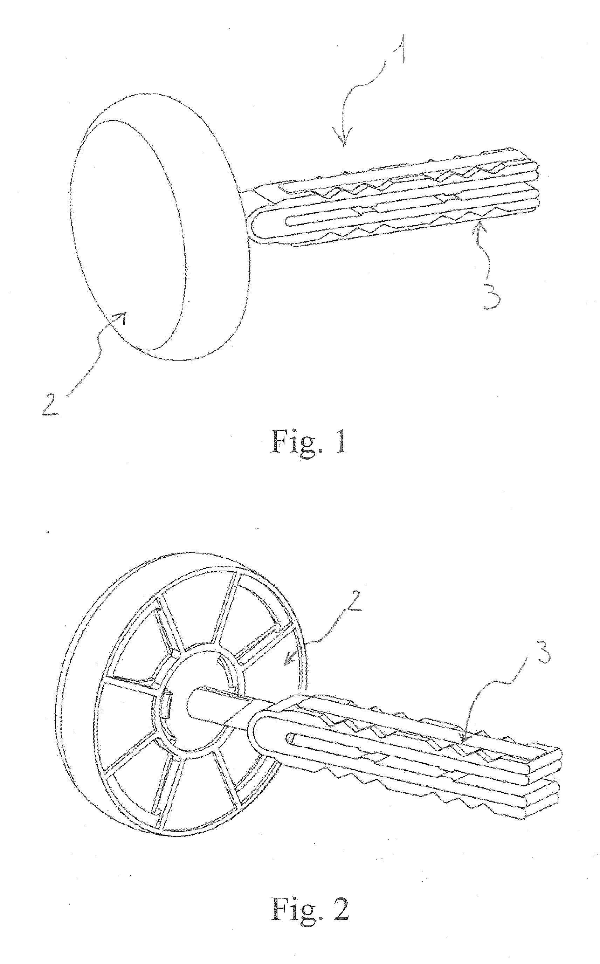

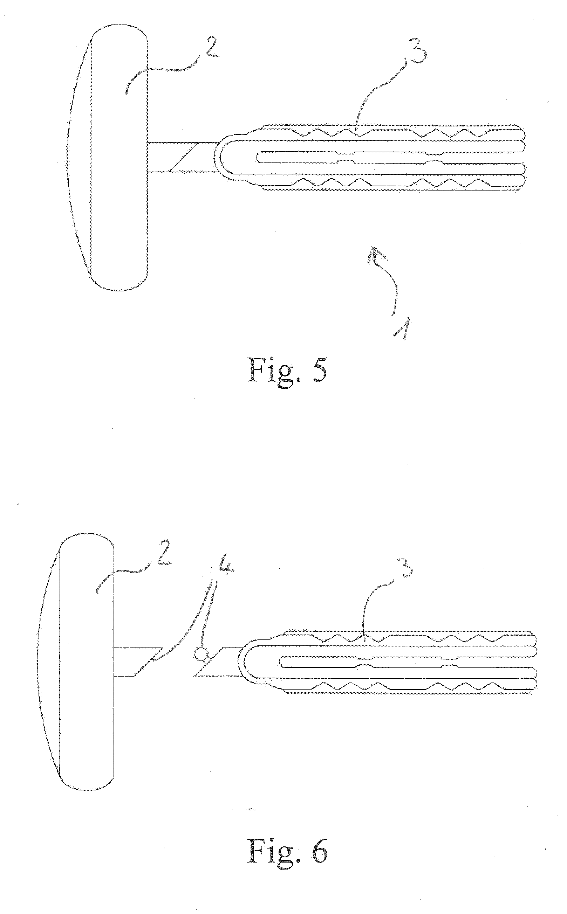

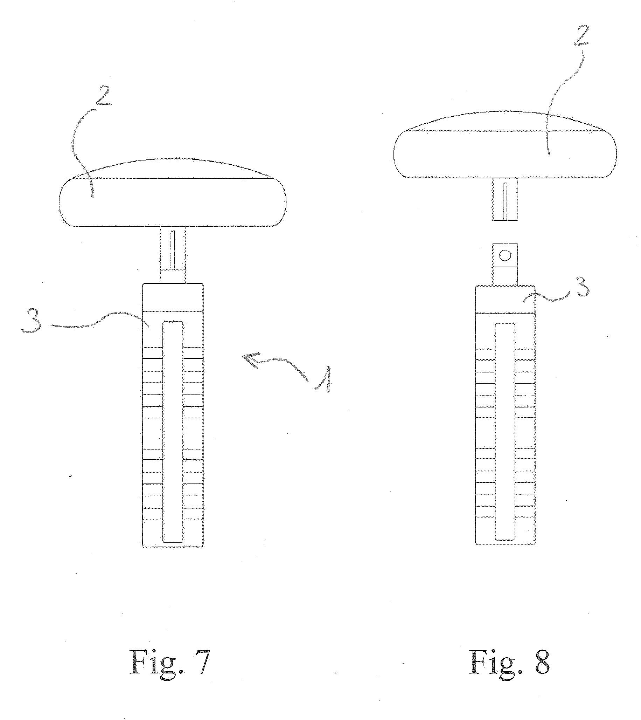

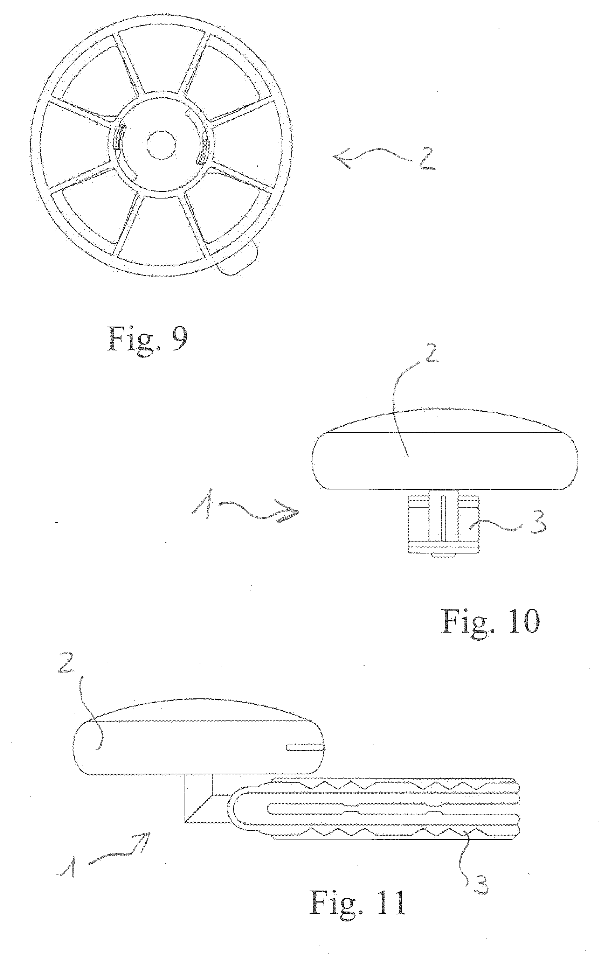

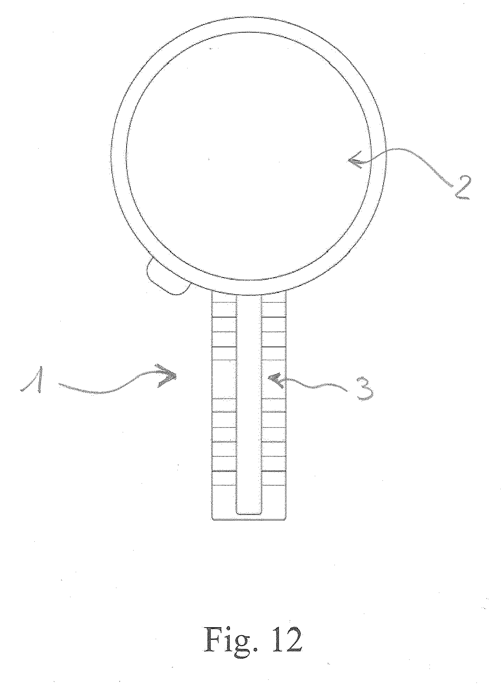

In accordance with the embodiment illustrated, the aforementioned connection means 4 comprise a spherical head in a spherical seat, so as to allow the support clip 3 to be positioned with respect to the container 2 so as to project perpendicularly from the container 2 (cf. figures 1, 2, 5 and 7) or according to a direction that is rotated by 90° (cf. figures 11 and 12), such two possible different arrangements being determined by the 45° cut between the head ends in contact with one another.

Preferably, the ambient fragrance dispenser 1 is provided already packed inside an air-tight sealed packaging (not illustrated). Such a packaging is impermeable to the passage of the second fragrancing agent that impregnates the first portion of said body so as to prevent the diffusion outside of said package of said second fragrancing agent all the time that said packaging is sealed air-tight.

Alternatively, it is possible to foresee a package in which the aforementioned air-tight sealed packaging that is impermeable to the passage of the second fragrancing agent is limited only to the aforementioned first portion 3 of the body of the ambient fragrance dispenser 1.

As a further alternative, the first portion 3 can be packed and sold individually from the rest of the ambient fragrance dispenser 1, in particular from the container 2 with the first fragrancing agent.

In accordance with the invention, the method for fragrancing an area with the ambient fragrance dispenser 1 considered above comprises the step of breaking the integrity of said packaging and arranging the ambient fragrance dispenser 1 in an area to be fragranced. Furthermore, such a method for fragrancing an area with the fragrance dispenser 1 moreover comprises the step of activating the aforementioned diffuser means that can be activated so as to allow the first fragrancing agent to be diffused in the environment outside the container:
- when the aforementioned packaging is being opened, so as to obtain the simultaneous and synergic fragrancing of the area both with the first fragrancing agent and with the second fragrancing agent thus exploiting a combination of two different perfumes/fragrances, or possibly of a perfume and an odour neutraliser, or, according to an alternative method, or
- with a time delay with respect to the instant in which the aforementioned packaging is opened, so as to initially have just the deodorant effect of the second fragrancing agent and, only after, have the fragrancing effect of the first fragrancing agent.

Preferably, the aforementioned diffuser means that can be activated to allow the first fragrancing agent to be diffused in the environment outside the container 3 are activated substantially close to when the fragrancing effect of the second fragrancing agent has run out.

In accordance with a different preferred embodiment, the aforementioned diffuser means that can be activated so as to allow the first fragrancing agent to be diffused in the area outside the container 3 are activated after the fragrancing effect of the second fragrancing agent has decreased by at least 25%, preferably after decreasing by at least 35%, more preferably after decreasing by at least 40%.

As can be appreciated from the present description, the ambient fragrance dispenser according to the present invention, as well as the package and the methods described, make it possible to satisfy the aforementioned requirements and to overcome, at the same time, the drawbacks referred to in the introduction of the present description with reference to the prior art. Indeed, the presence of the two different fragrancing agents and, especially, their different diffusion modalities makes it advantageously possible to use the same fragrance dispenser with different modalities so as to achieve different effects. For example for:
- modifying or integrating the fragrance emanated from the fragrance dispenser, in particular the fragrance of the second fragrancing agent of which the first portion 3 of the ambient fragrance dispenser 1 is impregnated;
- making it possible to activate the fragrance dispenser with the first fragrancing agent only after the fragrancing effect of the second fragrancing agent has run out;
- providing an effective "booster" effect, i.e. a more powerful fragrancing effect by using two different fragrancing technologies and/or methods, making it possible to use ambient fragrance dispensers provided with a container 2 for the first fragrancing agent having smaller dimensions considering the smaller amount of first fragrancing agent to be contained, which is particularly appreciated in fragrance dispensers for automobiles;
- obtaining a prolonged fragrance over time thanks to the possibility of carrying out the separated activation of the different fragrancing methods;
- obtaining a new fragrance by combining the two different fragrancing agents each having different fragrances.

Another advantage of the ambient fragrance dispenser according to the present invention lies in its structural simplicity.

In the case in which the fragrance dispenser device is used together with an air-vent, for example of an automobile or of a home air conditioning system, the simultaneous presence of the two different fragrancing methods of the area makes it possible to have a first fragrancing method that operates also when the ventilation is deactivated and the other fragrancing method activated (or rather activated more effectively) when the ventilation is activated through the aforementioned air outlet.

Of course, with the purpose of satisfying contingent and specific requirements, a man skilled in the art may carry out numerous modifications and variants to the ambient fragrance dispenser described above, all moreover covered by the field of protection of the invention as defined by the following claims.

## Claims

1. Ambient fragrance dispenser comprising:
- a body that comprises a container (2) containing a first fragrancing agent and
- diffuser means to allow said first fragrancing agent to be diffused from said container (2) towards the area outside of said body,
**characterised in that** at least one first portion (3) of said body is impregnated with a second fragrancing agent and is in communication with the area outside of said body, said second fragrancing agent of said fragrance dispenser being released by said first portion (3) of said body to be diffused towards the area surrounding said body independently from the diffusion of said first fragrancing agent.

2. Ambient fragrance dispenser according to claim 1, wherein said first portion (3) of said body is an outer portion of said body.

3. Ambient fragrance dispenser according to claim 1 or 2, wherein said first portion (3) of said body comprises a polymeric material impregnated with said second fragrancing agent, preferably a plastic polymeric material, more preferably a plastic polymeric material comprising EVA, PVC and/or polypropylene.

4. Ambient fragrance dispenser according to claim 1 or 2, wherein said first portion (3) of said body comprises a layer of cellulose impregnated with said second fragrancing agent.

5. Ambient fragrance dispenser according to any one of claims 1 to 4, wherein said first portion (3) of said body constitutes a support, gripping, clamping and/or fastening portion of said body.

6. Ambient fragrance dispenser according to any one of claims 1 to 5, wherein said first portion (3) of said body is fixed to the remaining part of said body through connection means (4), preferably removable snap-in connection means.

7. Ambient fragrance dispenser according to any one of claims 1 to 6, wherein said diffuser means are operatively associated with said container (2) and can be activated to pass from a deactivated condition, in which they prevent said first fragrancing agent from diffusing outside of said container (2), to an activated operative condition, in which they place the inside of said container (2) in communication with the outside to allow said first fragrancing agent to diffuse into the area outside said container (2).

8. Ambient fragrance dispenser according to claim 7, wherein said diffuser means comprise passages to place the inside of said container (2) in communication with the area outside said container (2) and partializing means applied to said passages, said partializing means being adjustable between an open position and a closed position.

9. Ambient fragrance dispenser according to any one of claims 1 to 8, wherein said diffuser means comprise membrane means permeable to said first fragrancing agent to allow a calibrated release of said first fragrancing agent with the area outside said container (2).

10. Ambient fragrance dispenser according to any one of claims 1 to 9, wherein:
- said container (2) is made in a single piece with said body or
- said container (2) defines an interchangeable capsule housed and/or supported in said body in a removable and/or interchangeable manner.

11. Ambient fragrance dispenser according to any one of claims 1 to 10, wherein said first fragrancing agent is in liquid, partially liquid or gel state.

12. Ambient fragrance dispenser according to any one of claims 1 to 11, wherein said first portion (3) of said body defines an outer portion of a membrane-type ambient fragrance dispenser, wherein the membrane of said membrane-type ambient fragrance dispenser allows the diffusion of the first fragrancing agent to be adjusted from said container (2) towards the area outside the container (2) itself.

13. Ambient fragrance dispenser according to any one of claims 1 to 12, wherein said second fragrancing agent is different from said first fragrancing agent in composition and/or fragrance.

14. **Package** comprising an air-tight sealed packaging inside which an ambient fragrance dispenser (1) according to any one of claims 1 to 13 is contained, said packaging being impermeable to the passage of said second fragrancing agent of said first portion (3) of said body to prevent the diffusion outside of said package of said second fragrancing agent all the time that said packaging is sealed air-tight.

15. **Method for making an ambient fragrance dispenser** made from polymeric material, comprising the steps of:
- providing a polymeric material in granular form;
- supplying and melting said granular polymeric material to obtain a molten polymer;
- by injection, moulding and/or hot extrusion of said molten polymer, making one or more portions of a body of an ambient fragrance dispenser (1) comprising a container (2) and
- inserting a first fragrancing agent into the container (2) of said body of the ambient fragrance dispenser (1),
**characterised in that** the granular polymeric material intended to constitute at least one first portion (3) of said body is subjected to a step of perfuming with a second fragrancing agent different from said first fragrancing agent, so as to obtain, by injection, moulding and/or hot extrusion of said polymeric material incorporating said second fragrancing agent inside it, said first portion of polymeric material of said body impregnated with said second fragrancing agent.

16. Method according to claim 15, wherein said step of perfuming said first portion (3) of said body with said second fragrancing agent different from said first fragrancing agent is performed by impregnation of said first portion (3) with said second fragrancing agent.

17. **Method for ambient fragrancing** through an ambient fragrance dispenser (1) that:
- comprises a body comprising a container (2) in which a first fragrancing agent is received,
- is equipped with diffuser means that can be activated to allow said first fragrancing agent to be diffused into the area outside said container (2),
- wherein at least one portion of said body of said ambient fragrance dispenser (1) is impregnated with a second fragrancing agent,
said ambient fragrance dispenser (1) being received in an air-tight sealed packaging, said packaging being impermeable to the passage of said second fragrancing agent that impregnates said first portion (3) of said body,
**said method comprising** the step of breaking the integrity of said packaging and arranging said ambient fragrance dispenser (1) in an area to be fragranced, so as to make said second fragrancing agent diffuse, which impregnates said first portion of the body in said area to be fragranced, also comprising the step of activating said diffuser means that can be activated to allow said first fragrancing agent to be diffused into the area outside said container (2):
- when said packaging is opened, so as to obtain the fragrancing of the area both with the first fragrancing agent and with the second fragrancing agent or
- with a time delay with respect to the opening of said packaging, so as to initially have just the deodorant effect of said second fragrancing agent and, only after, have the fragrancing effect of said first fragrancing agent.

18. Method for fragrancing an area according to claim 17, wherein said diffuser means that can be activated to allow said first fragrancing agent to be diffused into the area outside said container (2) are activated substantially close to when the fragrancing effect of said second fragrancing agent has run out.
